Europäisches Patentamt
European Patent Office
Office européen des brevets

(19)

(11) Veröffentlichungsnummer: **0 187 619**
**A2**

(12)
# EUROPÄISCHE PATENTANMELDUNG

(21) Anmeldenummer: 85810610.7

(22) Anmeldetag: 23.12.85

(51) Int. Cl.⁴: C 07 D 401/06
C 07 D 211/78

(30) Priorität: 03.01.85 CH 13/85

(43) Veröffentlichungstag der Anmeldung:
16.07.86 Patentblatt 86/29

(84) Benannte Vertragsstaaten:
AT BE CH DE FR GB IT LI LU NL SE

(71) Anmelder: CIBA-GEIGY AG
Klybeckstrasse 141
CH-4002 Basel(CH)

(72) Erfinder: Fröstl, Wolfgang, Dr.
Johanns-Vorstadt 6/2
CH-4056 Basel(CH)

(72) Erfinder: Storni, Angelo, Dr.
Im Feuerbusch 3
CH-4310 Rheinfelden(CH)

(54) **1,3-disubstituierte Tetrahydropyridine.**

(57) 1-(3-Indolyl)-tetrahydropyridine der Formel

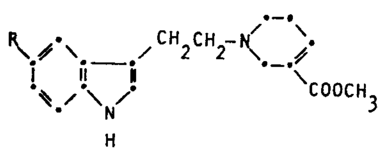

(I)

worin R Wasserstoff oder Methoxy bedeutet, haben nootrope Wirkung und können als Nootropika Verwendung finden. Sie werden beispielsweise hergestellt, indem man Verbindungen der Formeln

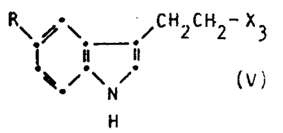

(V) und

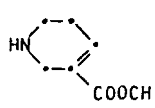

(VI),

worin $X_3$ eine nukleofuge Abgangsgruppe bedeutet, oder deren Salze miteinander umsetzt.

EP 0 187 619 A2

0187619

CIBA-GEIGY AG
Basel (Schweiz)

4-15214/+

1,3-disubstituierte Tetrahydropyridine

Die Erfindung betrifft neue 1-(3-Indolyl)-tetrahydro-pyridin-3-carbonsäureester der Formel

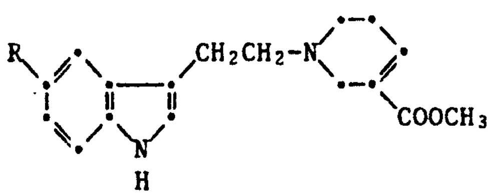

(I)

worin R Wasserstoff oder Methoxy bedeutet, und ihre Salze, insbesondere pharmazeutisch verwendbare Salze.

Salze von Verbindungen der Formel I sind beispielsweise deren pharmazeutisch verwendbare Säureadditionssalze.

Pharmazeutisch verwendbare Säureadditionssalze sind beispielsweise Salze mit geeigneten Mineralsäuren, wie Halogenwasserstoffsäuren, Schwefelsäure oder Phosphorsäure, z.B. Hydrochloride, Hydrobromide, Sulfate, Hydrogensulfate oder Phosphate, oder Salze mit geeigneten organischen Carbon- oder Sulfonsäuren, wie gegebenenfalls hydroxylierten aliphatischen Mono- oder Dicarbonsäuren, z.B. Acetate, Oxalate, Succinate, Fumarate, Maleinate, Maleate, Ascorbinate oder Citrate, oder aliphatischen oder aromatischen Sulfonsäuren oder N-substituierten Sulfaminsäuren, z.B. Methansulfonate, Benzolsulfonate, p-Toluolsulfonate oder N-Cyclohexylsulfaminate (Cyclamate).

Zur Isolierung oder Reinigung können auch pharmazeutisch ungeeignete Salze Verwendung finden. Zur therapeutischen Anwendung gelangen nur die pharmazeutisch verwendbaren, nicht-toxischen Salze, die deshalb bevorzugt sind.

Im Stand der Technik sind einige Isomere der Verbindung der Formel I, worin R Wasserstoff bedeutet, bekannt. So wird in J. Am. Chem. Soc. 87, Seiten 5461-5467 (1965) 1-[2-(Indol-3-yl)-ethyl]-1,4,5,6-tetrahydro-pyridin-3-carbonsäuremethylester (Isomeres A) als Zwischenprodukt für die Synthese des Alkaloides Dihydrocorynantheol vorgeschlagen. Ferner ist in J. Am. Pharm. Ass. 40, Seiten 589-590 (1951) 1-(Indol-3-yl)methyl-1,2,5,6-tetrahydro-pyridin-3-carbonsäureethylester (Isomeres B) beschrieben und unter Bezugnahme auf eine behauptete strukturelle Verwandtschaft zu dem Wehenmittel Ergonovin zur Untersuchung auf mögliche ähnliche oxytoxin-artige Eigenschaften vorgeschlagen worden. Weiterhin ist in J. Chem. Soc.(C), Seiten 736-743 (1971) 1-[2-(Indol-3-yl)ethyl]-1,2,5,6-tetrahydro-pyridin-4-carbonsäuremethylester (Isomeres C) als mögliches Zwischenprodukt von 2-Methoxycarbonyl-octahydro-indolo[2,3-a]chinolizin beschrieben, dessen Grundgerüst sich in einigen Alkaloiden findet. Die Verbindungen der Formel I sind demgegenüber neu. Im Stand der Technik finden sich auch keinerlei Anhaltspunkte für eine mögliche Wirkung von 1-(Indol-3-yl-alkyl)-tetrahydropyridin-3-carbonsäureverbindungen auf das zentrale Nervensystem. Der Erfindung liegt die überraschende Feststellung zugrunde, dass die Verbindungen der Formel I und ihre pharmazeutisch verwendbaren Salze wertvolle pharmakologische Wirkungen auf das zentrale Nervensystem, insbesondere ausgeprägte nootrope Eigenschaften aufweisen.

So vermindern sie an der Maus in Dosen ab etwa 0,1 mg/kg i.p. sowie p.o. die amnesiogene Wirkung eines Elektroschocks in mindestens gleichem Ausmass wie nach Verabfolgung einer nootrop wirksamen Dosis von Piracetam (100 mg/kg i.p.). Zum Nachweis der nootropen Wirkung kann beispielsweise der Two-Compartment-Test herangezogen werden. Als Literatur über pharmakologische Modelle dieser Art seien z.B.

genannt: S.J. Sara und D. Lefevre, Psychopharmacologia 25, 32-40 (1972), Hypoxia-induced amnesia in one-trial learning and pharmacological protection by piracetam. Boggan, W.O., und Schlesinger, K., in Behavioral Biology 12, 127-134 (1974). In diesem Modell zeigt 1-[2-(Indol-3-yl)ethyl]-1,2,5,6-tetrahydro-pyridin-3-carbonsäureester in Dosen von 0,1 und 1,0 mg/kg ip. einen hochsignifikanten ($p<0,01$) antiamnesiogenen Effekt. Das Isomere B zeigt bei 0,1, 1,0 und 10 mg/kg i.p. einen ähnlichen, jedoch weniger signifikanten ($p<0,05$) Effekt. Das Isomere A zeigt lediglich bei 1,0 mg/kg einen gewissen (nur einseitig signifikanten), bei 0,1 und 10 mg/kg keinen signifikanten, insgesamt somit unsicheren Effekt. Das Isomere C zeigt bei 0,1, 1,0 und 10 mg/kg i.p. keine signifikante antiamnesiogene Wirkung.

Insbesondere zeigen die Verbindungen der Formel I eine starke gedächtnisverbessernde Wirkung im Step-down Passive Avoidance Test nach Mondadori und Waser, Psychopharmacology 63, 297-300 (1979). Die Substanzen sind wirksam bei intraperitonealer Gabe 30 Minuten vor dem Lernversuch (wirksame Dosen 0,1, 1, 10 mg/kg). Eine deutliche Wirkung war auch feststellbar bei peroraler Applikation 60 Minuten vor dem Lernversuch (wirksame Dosen 0,1, 1, 10 mg/kg) sowie bei intraperitonealer Applikation unmittelbar nach dem Lernversuch (wirksame Dosen 0,1, 1, 10 mg/kg). In diesem Modell bewirkt 1-[2-(Indol-3-yl)ethyl]-1,2,5,6-tetrahydro-pyridin-3-carbonsäuremethylester bei 0,1 und 1,0 mg/kg i.p. eine signifikante ($p<0,05$) und bei 10 mg/kg i.p. eine hochsignifikante ($p<0,01$) Gedächtnisverbesserung, wohingegen die Isomeren A, B und C sämtlichst bei 0,1 und 1,0 mg/kg ip. keinen und erst bei 10 mg/kg ip. einen signifikanten ($p<0,05$) Effekt zeigen. Daraus ergibt sich, dass, um die gedächtnisverbessernde Wirkung von 0,1 mg/kg i.p. von 1-[2-(Indol-3-yl)ethyl]-1,2,5,6,-tetrahydro-pyridin-3-carbonsäuremethylester zu erzielen, 10 mg/kg i.p., d.h. die 100-fache Dosis eines Isomeren A, B und C benötigt werden. Die Signifikanzangaben beziehen sich, ebenso wie im vorhergehenden Absatz, auf den U-Test von Mann und Whitney.

Vor allem auf Grund der letzgenannten Befunde werden die Verbindungen der Formel I und ihre pharmazeutisch verwendbaren Salze für die Verwendung als Nootropika, beispielsweise zur Behandlung zerebraler Leistungsinsuffizienz, insbesondere von Gedächtnisstörungen unterschiedlicher Genese, wie senile Demenz oder Demenz vom Alzheimer-Typ, ferner von Folgezuständen von Hirntraumata und Apoplexien, vorzüglich geeignet angesehen. Hinzu kommt noch ihre ausgezeichnete Verträglichkeit.

Die Erfindung betrifft namentlich die in den Beispielen genannten Verbindungen der Formel I und ihre pharmazeutisch verwendbaren Salze, insbesondere Säureadditionssalze, wie ihre Hydrochloride oder Hydrobromide.

Die Erfindung betrifft ferner ein auf an sich bekannten Synthesemethoden beruhendes Verfahren zur Herstellung von Verbindungen der Formel I unter Einschluss ihrer Salze.

Das erfindungsgemässe Verfahren ist dadurch gekennzeichnet, dass man

a) in einer Verbindung der Formel

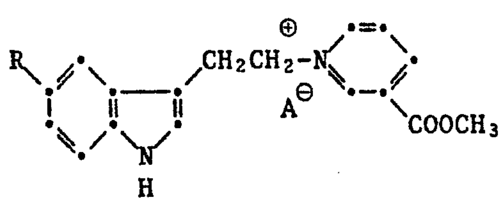

(II),

worin $A^{\ominus}$ ein Säureanion bedeutet, die überschüssigen Doppelbindungen zu Einfachbindungen reduziert oder

b) aus einer Verbindung der Formel

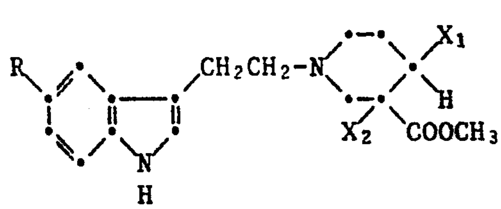

(III),

worin einer der Reste $X_1$ und $X_2$ Wasserstoff und der andere einen abspaltbaren Rest bedeutet, oder einem Salz davon, $X_1$ und $X_2$ unter Bildung der Doppelbindung abspaltet, oder

c) eine Verbindung der Formel

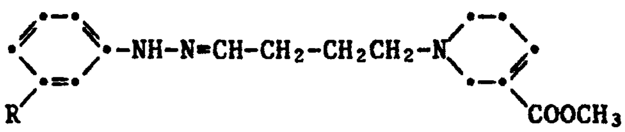 (IV)

oder ein Salz davon zu der entsprechenden Verbindung der Formel I ringschliesst, oder

d) Verbindungen der Formeln

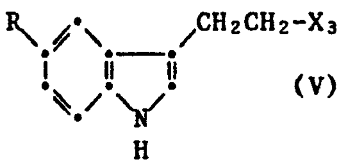 (V) und 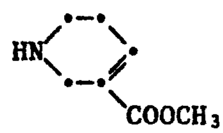 (VI),

worin $X_3$ eine nukleofuge Abgangsgruppe bedeutet, oder deren Salze miteinander umsetzt, oder

e) eine Verbindung der Formel

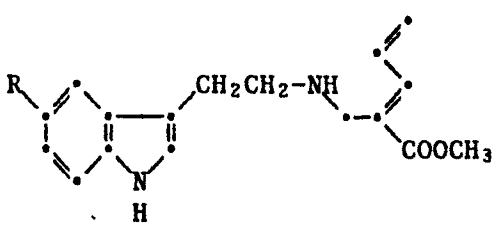 (VII),

oder ein Salz davon cyclisiert, oder

f) in einer Verbindung der Formel

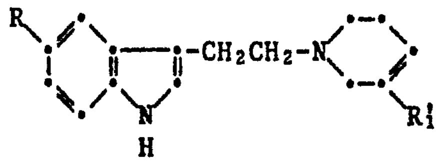

(VIII),

worin $R_1'$ einen in Methoxycarbonyl überführbaren Rest bedeutet, oder in einem Salz davon $R_1'$ in Methoxycarbonyl überführt, oder

g) zur Herstellung der Verbindung der Formel I, worin R Methoxy ist, in einer Verbindung der Formel

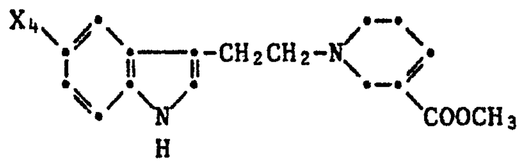

(IX),

worin $X_4$ einen in Methoxy überführbaren oder gegen Methoxy austauschbaren Rest bedeutet, $X_4$ in Methoxy überführt oder gegen Methoxy austauscht und gewünschtenfalls die verfahrensgemäss erhältliche Verbindung in eine andere Verbindung der Formel I überführt, ein verfahrensgemäss erhältliches Isomerengemisch in die Komponenten auftrennt und/oder eine verfahrensgemäss erhältliche freie Verbindung in ein Salz oder ein verfahrensgemäss erhältliches Salz in die freie Verbindung oder in ein anderes Salz umwandelt.

Verfahrensvariante a)

Das Anion $A^{\ominus}$ ist beispielsweise das Anion einer starken Protonensäure, die alkoholisches Hydroxy in reaktionsfähiges verestertes Hydroxy zu überführen vermag, z.B. ein Halogenidion, wie Chlorid, Bromid oder Iodid, oder ein Sulfonation, insbesondere das Anion einer aliphatischen oder aromatischen Sulfonsäure, z.B. das Methansulfonat-, Ethansulfonat- oder Methosulfato-ion bzw. das Benzolsulfonat-, p-Toluolsulfonat- oder p-Brombenzolsulfonation.

Die Reduktion der überschüssigen Doppelbindungen erfolgt in üblicher Weise, beispielsweise durch Einwirkung eines Dileichtmetallhydrides, wie eines Alkalimetall-triniederalkoxyaluminiumhydrides, z.B. von Lithium-tritertiärbutyloxyaluminiumhydrid, oder eines gegebenenfalls substituierten Alkalimetallborhydrides, wie von Lithiumborhydrid, Kaliumborhydrid, Natriumborhydrid oder Natriumcyanborhydrid. Die Reaktion wird vorzugsweise in einem dafür üblichen Lösungsmittel durchgeführt. Ueblich sind beispielsweise für die Umsetzung mit Lithium-tritertiärbutyloxyaluminiumhydrid etherartige Lösungsmittel, wie Diethylether, Dioxan oder Tetrahydrofuran,und für die Umsetzung mit gegebenenfalls substituierten Alkalimetallborhydriden insbesondere Niederalkanole, wie Methanol oder Ethanol, ferner Wasser.

Zwischenprodukte II können beispielsweise durch Umsetzung einer Verbindung der Formel

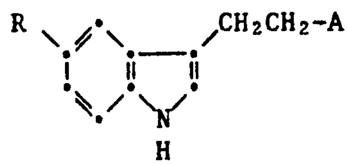

(XI),

worin A dem Anion $A^{\ominus}$ entsprechendes reaktionsfähiges verestertes Hydroxy bedeutet, mit Nikotinsäuremethylester erhalten werden.

Verfahrensvariante b)

In Zwischenprodukten III bedeutet der von Wasserstoff verschiedene Rest $X_1$ bzw. $X_2$ gegebenenfalls verestertes Hydroxy, wie Hydroxy, mit einer Sulfonsäure, wie Alkan- oder gegebenenfalls substituierten Benzolsulfonsäure, verestertes Hydroxy, z.B. Methan-, Ethan-, Benzol-, p-Toluol- oder p-Brombenzolsulfonyloxy, mit einer Carbonsäure verestertes Hydroxy, wie Niederalkanoyloxy, z.B. Acetoxy, oder Halogen, z.B. Chlor oder Brom, oder eine gegebenenfalls substituierte Amino-, Ammonio- oder Oxidoammoniogruppe, wie Amino, gegebenenfalls substituiertes Anilino, Mono- oder Diniederalkylamino, z.B. Mono- oder Dimethylamino, Triniederalkylammonio, z.B. Trimethylammonio, oder N,N-Diniederalkyl-, z.B. N,N-Dimethyl-N-oxido-ammonio.

Die Abspaltung von $X_1$ und $X_2$ erfolgt in üblicher Weise, ausgehend von Verbindungen III, worin einer der Reste $X_1$ und $X_2$ Niederalkanoyloxy oder eine gegebenenfalls substituierte Oxidoammoniogruppe, z.B. N,N-Diniederalkyl-N-oxido-ammonio, bedeutet, beispielsweise thermisch, z.B. im Temperaturbereich von etwa 60 bis etwa 150°C. Bedeutet der von Wasserstoff verschiedene Rest $X_1$ bzw. $X_2$ gegebenenfalls mit einer Carbonsäure verestertes Hydroxy oder gegebenenfalls substituiertes Amino, arbeitet man erforderlichenfalls in Gegenwart eines sauren Mittels, insbesondere einer Protonensäure, wie einer Mineralsäure, bzw. eines sauren Salzes derselben, z.B. von Chlor- oder Bromwasserstoffsäure, Schwefelsäure bzw. Natriumhydrogensulfat, Phosphorsäure oder Polyphosphorsäure, einer Niederalkansäure, z.B. Essigsäure, einer organischen Sulfonsäure, z.B. p-Toluolsulfonsäure, oder eines sauren Ionenaustauschers. Als Nebenprodukt wird dabei Wasser, die entsprechende Carbonsäure, Ammoniak bzw. das entsprechende Amin gebildet, das vorteilhaft physikalisch, z.B. durch Destillation bzw. azeotrope Destillation, oder chemisch, z.B. mittels eines wasserbindenden Mittels oder Bildung von Ammoniumsalzen, aus dem Reaktionsgemisch entfernt wird. Bedeutet der von Wasserstoff verschiedene Rest $X_1$ bzw. $X_2$ Triniederalkylammonio, mit einer Sulfonsäure verestertes Hydroxy oder Halogen, arbeitet man vorzugsweise in Gegenwart einer Base, wie des Hydroxydes, Carbonates oder eines Alkoholates eines Alkalimetalls, z.B. von Natrium- oder Kaliumhydroxid, Kaliumcarbonat oder Natriummethanolat, oder eines tertiären organischen Amins, z.B. von Diazabicyclononan oder Diazabicycloundecan.

Die Zwischenprodukte III können in an sich bekannter Weise hergestellt werden, wobei man Herstellung und Weiterumsetzung derselben mit Vorteil so durchführt, dass das Zwischenprodukt III ohne Isolierung weiterumgesetzt werden kann.

So kann man beispielsweise eine Verbindung der Formel

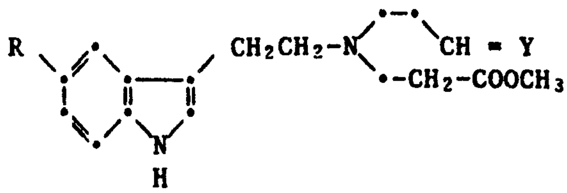

(XII),

worin Y Oxo oder gegebenenfalls substituiertes Imino bedeutet, der gleichzeitigen Behandlung mit einer Säure, insbesondere einer aliphatischen Carbonsäure, z.B. von Essigsäure, und einer Base, insbesondere eines aliphatischen Amins, wie eines Mono-, Di- oder Triniederalkylamins bzw. Niederalkylen- bzw. Aza-, Oxa- oder Thianiederalkylenamins, z.B. von Triethylamin, Piperidin oder Morpholin, unterwerfen. Dabei cyclisiert die Verbindung XII intermediär zu einem Zwischenprodukt III, worin $X_1$ Hydroxy oder gegebenenfalls monosubstituiertes Amino bedeutet, das unter den Reaktionsbedingungen zum Endprodukt I weiterreagiert. Die Umsetzung kann aber auch auf der Stufe des Zwischenproduktes III aufgehalten werden, indem man die Cyclisierung in Gegenwart einer Metallbase, wie eines Metallalkoholats, z.B. von Natriummethanolat oder -ethanolat, durchführt. Verbindungen XII werden ihrerseits z.B. erhalten, indem man einen reaktionsfähigen Indol-3-yl-alkanolester der Formel

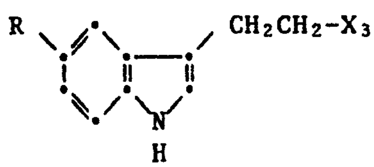

(V),

worin $X_3$ reaktionsfähiges verestertes Hydroxy ist, zunächst mit ß-Alaninmethylester und dann mit Acrolein oder einem gegebenenfalls funktionell abgewandelten Aldehyd der Formel $Y_1-CH_2-CH_2-CH=O$ (XIII; $Y_1$ = reaktionsfähiges verestertes Hydroxy) umsetzt.

Zwischenprodukte III, worin $X_1$ Triniederalkylammonio bedeutet, können beispielsweise durch erschöpfende Niederalkylierung von Verbindungen III, worin $X_1$ Amino bzw. Niederalkylamino bedeutet, erhalten werden, insbesondere durch Behandlung mit einem reaktionsfähigen Niederalkanolester, wie einem Niederalkylbromid oder -jodid

erhalten werden. Zwischenprodukte III, worin $X_1$ N-Diniederalkyl-N-oxido-ammonio ist, erhält man insbesondere durch N-Oxidation entsprechender Diniederalkylaminverbindungen (III, $X_1$ = Diniederalkylamino), z.B. mittels organischer Peroxyverbindungen.

Zwischenprodukte III, worin $X_1$ Hydroxy ist, können auch durch Reduktion einer entsprechenden Verbindung der Formel

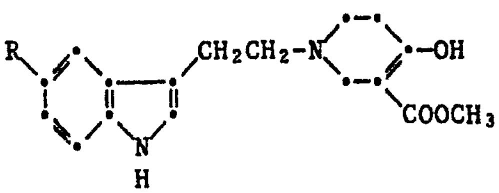

(XIV)

oder des entsprechenden 4-Oxotautomeren erhalten werden, beispielsweise durch katalytische Hydrierung, insbesondere von Platinoxid, oder durch Umsetzung mit einem Alkalimetall-triniederalkoxylithiumaluminiumhydrid oder Alkalimetallborhydrid, z.B. mit Lithium-tritertiärbutyloxyaluminiumhydrid, Natrium- oder Lithiumborhydrid oder Natriumcyanoborhydrid. Verbindungen XIV können ihrerseits hergestellt werden, indem man eine Verbindung der Formel

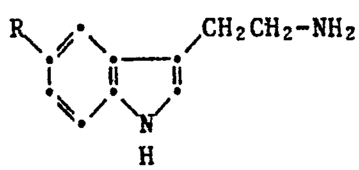

(XV)

mit der annähernd doppelt-molaren Menge einer Verbindung der Formel $Y_1-CH_2CH_2-COOCH_3$ (XVI; $Y_1$ = reaktionsfähiges verestertes Hydroxy) bzw. Acrylsäuremethylester umsetzt und das Reaktionsprodukt der Formel

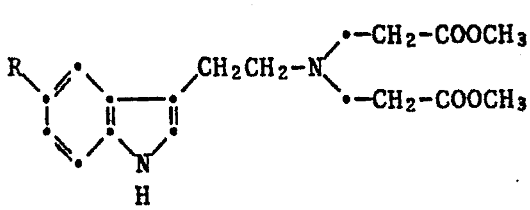

(XVIIa),

z.B. in Gegenwart einer Metallbase, wie eines Alkalimetallalkoholats, z.B. von Natriummethanolat, cyclisiert.

Zwischenprodukte III, worin $X_1$ verestertes Hydroxy ist, können beispielsweise durch Veresterung von entsprechenden Hydroxyverbindungen erhalten werden, indem man diese mit dem Anhydrid oder einem Halogenid einer Sulfon- oder Carbonsäure, wie einem Alkan- oder gegebenenfalls substituierten Benzolsulfonylchlorid, z.B. mit Methan-, Ethan-, Benzol-, p-Toluol- oder p-Brombenzolsulfonylchlorid oder einem Niederalkansäureanhydrid oder -halogenid, z.B. mit Acetanhydrid, oder mit Thionylchlorid oder Phosphortribromid umsetzt. Arbeitet man dabei in Gegenwart einer Base, z.B. von Kaliumcarbonat, Triethylamin oder Pyridin, kann die Reaktion so geführt werden, dass der gebildete Ester III direkt zum entsprechenden Endprodukt I weiterreagiert.

Zwischenprodukte III, worin $X_2$ Halogen ist, können z.B. erhalten werden, indem man eine Verbindung der Formel

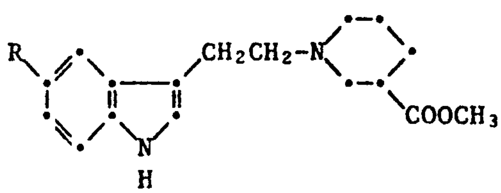

(XVIII),

zugänglich durch Umsetzung einer Verbindung der Formel

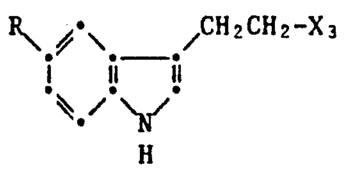

(V),

worin $X_3$ reaktionsfähiges verestertes Hydroxy ist, mit Piperidin-3-carbonäuremethylester, in üblicher Weise in α-Stellung zur Methoxycarbonylgruppe halogeniert, z.B. durch Behandeln mit Chlor oder Brom bzw. mittels N-Chlor- oder N-Bromsuccinimid.

Reaktionsfähiges verestertes Hydroxy ist dabei insbesondere Halogen, wie Chlor, Brom oder Iod.

Verfahrensvariante c)

Die Umlagerung, bei der im Sinne der Fischer'schen Indolsynthese Ammoniak abgespalten wird, kann in üblicher Weise erfolgen, beispielsweise durch Säurebehandlung, d.h. Einwirkung einer geeigneten Protonen- oder Lewissäure. Als Protonensäuren kommen beispielsweise Mineralsäuren, wie Schwefelsäure, Phosphorsäure, ebenso Chlorwasserstoff in einem Niederalkanol, wie ethanolische Salzsäure, und organische Sulfonsäuren, wie eine Niederalkan-, z.B. Methansulfonsäure, oder gegebenenfalls substituierte Benzolsulfonsäuren, wie Benzol-oder p-Toluolsulfonsäure, aber auch organische Carbonsäuren, wie Niederalkansäuren, z.B. Essigsäure, in Betracht. Als Lewissäuren sind beispielsweise koordinativ ungesättigte Schwermetallverbindungen, wie koordinativ ungesättigte Halogenide des Bors, Aluminium, Antimons, Zinks, Kupfers, Nickels, Eisens, Chroms oder Zinns, z.B. Zinkchlorid oder Kupfer-I-chlorid bzw. -bromid, geeignet. Die Umsetzung wird vorteilhaft unter Erwärmen, z.B. im Temperaturereich von etwa 60 bis 170°C, erforderlichenfalls unter Inertgas, durchgeführt.

Zwischenprodukte IV können z.B. hergestellt werden, indem man ein gegebenenfalls entsprechend substituiertes Phenylhydrazin in üblicher Weise mit 4-[1-(3-Methoxycarbonyl-1,2,5,6-tetrahydropyridyl)]-butyraldehyd kondensiert.

Verfahrensvariante d)

Nukleofuge Abgangsgruppen $X_3$ sind beispielsweise reaktionsfähige veresterte Hydroxygruppen, wie Halogen, z.B. Chlor, Brom oder Iod, oder von Sulfonsäuren, wie Niederalkan- oder gegebenenfalls substituierten Benzolsulfonsäuren, oder Halogensulfonsäuren abgeleitete Sulfonyloxygruppen, z.B. Methansulfonyloxy, Benzolsulfonyloxy, p-Toluolsulfonyloxy, p-Brombenzolsulfonyloxy oder Fluorsulfonyloxy.

Die Umsetzung von Verbindungen V und VI erfolgt in üblicher Weise, beispielsweise in Gegenwart eines basischen Kondensationsmittels, wie einer tertiären organischen Stickstoffbase, z.B. von Pyridin

oder eines Triniederalkylamins, wie von Triethylamin. Man kann aber auch Alkalimetallhydroxide oder -carbonate, z.B. Natriumhydroxid, verwenden, erforderlichenfalls in Gegenwart von Phasenübergangsvermittlern (phase transfer catalyst), z.B. von Benzyl-trimethyl-ammoniumhydroxid.

Ausgangsstoffe V können z.B. hergestellt werden, indem man ein entsprechendes Indol mit einem 1,2-Dihalogenethan bzw. 2-Halogenethanol kondensiert bzw. mittels Oxalylchlorid in das entsprechende 3-Chloroxalylindol überführt, dieses z.B. mit Ethanol, zum entsprechenden 3-Ethoxyoxalylindol alkoholisiert und den erhaltenen Oxalsäureester reduziert, z.B. mittels Lithiumaluminiumhydrid in Diethylether, und das gegebenenfalls erhaltene 2-(3-Indolyl)-ethanol anschliessend reaktionsfähig verestert, z.B. durch Umsetzung mit einem Halogenierungs- oder Sulfonylierungsmittel, z.B. mit Thionylchlorid, Phosphortribromid oder einem Sulfonsäurechlorid, wie p-Toluolsulfonylchlorid.

Ausgangsstoffe VI werden z.B. erhalten, indem man Arecolin durch Behandeln mit einem Chlorameisensäurealk(en)ylester entmethyliert und das gebildete 1-Alk(en)yloxycarbonylderivat von VI zu diesem hydrolysiert. Man kann aber auch 6-(p-Toluolsulfonyloxyimino)-hex-2-en-carbonsäuremethylester mit Diethylaluminiumchlorid zum entsprechenden 5,6-Dihydropyridin-3-carbonsäuremethylester umlagern und dieses mit Natriumborhydrid zum 1,2,5,6-Tetrahydroanalogen reduzieren.

<u>Verfahrensvariante e)</u>
Die Cyclisierung von Verbindungen VII erfolgt spontan oder durch Basenbehandlung, wobei als Basen beispielsweise Alkalimetallalkanolate, -amide oder -hydride, z.B. Natriummethanolat, Kaliumtertiärbutanolat, Natriumamid, Lithium-N,N-diisopropylamid oder Natriumhydrid, verwendet werden können.

Die Zwischenprodukte VII werden vorteilhaft in situ hergestellt und ohne Isolierung erfindungsgemäss weiterumgesetzt, indem man 2-Brommethyl-penta-2,4-dien-carbonsäuremethylester in Gegenwart einer Base, wie einer der genannten, z.B. von Natriumhydrid, oder eines Alkalimetallhydroxides oder -carbonates, z.B. von Natriumhydroxid oder Kaliumcarbonat, mit einer Verbindung der Formel

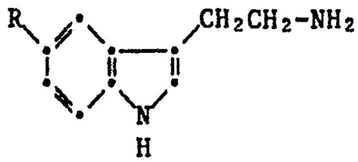

(XV)

umsetzt, z.B in Dimethylformamid, N-Methylpyrrolidon oder Dimethylsulfoxid.

Die dafür notwendigen Ausgangsstoffe werden z.B. erhalten, indem man ein 2-(5-R-Indol-3-yl)-acetonitril, z.B. mit Lithiumaluminiumhydrid in Diethylether oder Tetrahydrofuran, reduziert oder eine Verbindung der Formel

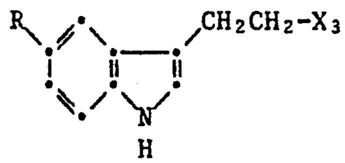

(V),

worin $X_3$ z.B. Brom ist, mit Ammoniak umsetzt bzw. Acrolein mit einem 2-Triphenylphosphorylenpropionsäuremethylester kondensiert und in dem erhaltenen 2-Methyl-penta-2,4-dien-carbonsäuremethylester die 2-Methylgruppe, z.B. mittels N-Bromsuccinimid, bromiert.

Verfahrensvariante f)

Der in Methoxycarbonyl überführbare Rest $R_1'$ ist beispielsweise Carboxy, anhydridisiertes Carboxy, von Methoxycarbonyl verschiedenes verestertes Carboxy, Trimethoxymethyl, Methoxycarbimino der Formel $-C(=NH)-OCH_3$ oder Cyano. Anhydridisiertes Carboxy ist beispielsweise Halogencarbonyl, wie Chlorcarbonyl. Von Methoxycarbonyl verschiedenes verestertes Carboxy ist beispielsweise mit einem aliphatischen, araliphatischen oder aromatischen Alkohol (ausser Methanol) verestertes Carboxy, wie Niederalkoxycarbonyl (ausser Methoxycarbonyl), z.B. Ethoxy-, Propyloxy-, Isopropyloxy- oder Tertiärbutyloxycarbonyl, Niederalkenyloxycarbonyl, z.B. Vinyloxycarbonyl, Phenylniederalkoxycarbonyl, z.B. Benzyloxycarbonyl, oder gegebenenfalls substituiertes Phenoxycarbonyl, z.B. Phenoxy-, p-Nitrophenoxy- oder 2,4-Dinitrophenoxycarbonyl.

Die Ueberführung der genannten Gruppen $R_1'$ in Methoxycarbonyl erfolgt in üblicher Weise, beispielsweise durch Hydrolyse oder Methanolyse. So kann man Trimethoxymethyl bzw. Methoxycarbimino durch Hydrolyse unter sauren Bedingungen, Carboxy durch Ueberführung in eine Salz-, z.B. Alkalimetallsalzform, und Umsetzung mit einem Methylierungsmittel, z.B. mit Methyljodid oder Dimethylsulfat, oder insbesondere Umsetzung mit Methanol unter sauren Bedingungen, anhydridisiertes Carboxy durch Umsetzung mit Methanol unter basischen Bedingungen, von Methoxycarbonyl verschiedenes verestertes Carboxy durch Umsetzung mit Methanol unter sauren oder basischen Bedingungen sowie Cyano durch Umsetzung mit Methanol in Gegenwart von Wasser unter sauren Bedingungen zu Methoxycarbonyl solvolysieren.

Solvolysereaktionen unter sauren Bedingungen werden beispielsweise in Gegenwart eines sauren Mittels, wie einer Protonensäure, beispielsweise einer Mineralsäure, wie Schwefelsäure, Phosphorsäure oder einer Halogenwasserstoffsäure, z.B. Chlor- oder Bromwasserstoffsäure, bzw. eines sauren Salzes einer solchen, z.B. von Ammoniumchlorid, oder einer organischen Sulfonsäure, z.B. von p-Toluolsulfonsäure, erforderlichenfalls in Gegenwart eines Lösungs- oder Verdünnungsmittels, z.B. eines mit Wasser bzw. mit Methanol mischbaren Lösungsmittels, und/oder unter Erwärmen, z.B. im Temperaturbereich von etwa 20° bis etwa 120°C, z.B. bei ungefährer Siedetemperatur, d.h. etwa 60° bis 70°, bzw. 90° bis 105°C, durchgeführt.

Bei Solvolysereaktionen unter basischen Bedingungen setzt man die Reaktionskomponente VIII beispielsweise mit einem Alkalimetall- oder Erdalkalimetallalkoholat, z.B. mit Natriummethanolat um. Die

basische Methanolyse von anhydridisierten oder veresterten Carboxygruppen kann aber auch in Gegenwart eines Alkalimetallhydroxides, z.B. von Kaliumhydroxid, die basische Methanolyse von anhydridisiertem Carboxy auch in Gegenwart eines Alkalimetallcarbonates, z.B von Natrium- oder Kaliumcarbonat, oder einer organischen Stickstoffbase wie eines Triniederalkyl- oder sterisch gehinderten Diniederalkylamins, z.B. von Triethylamin oder Diisopropylamin, oder einer tertiären aromatischen Stickstoffbase, z.B. von Pyridin, erfolgen. Erforderlichenfalls arbeitet man in Gegenwart eines Lösungs- oder Verdünnungsmittels, wie eines mit Wasser bzw. Methanol mischbaren Lösungsmittels, und/oder unter Kühlen oder Erwärmen, z.B. im Temperaturbereich von etwa -50 bis +110°C.

In einer bevorzugten Ausführungsform der Verfahrensvariante f) wird Carboxy $R_1'$ durch Veresterung, beispielsweise durch Umsetzung mit Methanol in Gegenwart einer Mineralsäure, wie Schwefelsäure oder Chlorwasserstoffsäure, durch Ueberführung in ein Metallsalz, insbesondere Alkalimetallsalz und anschliessende Umsetzung mit einem Methylierungsmittel, beispielsweise einem reaktionsfähigen Ester, d.h. einem Halogenwasserstoffsäure-, Schwefelsäure- oder Sulfonsäureester des Methanols, z.B. mit Methyljodid bzw. Methylbromid, Dimethylsulfat, p-Toluolsulfonsäuremethylester oder Fluorsulfonsäuremethylester, oder durch Umsetzung mit Diazomethan in Methoxycarbonyl überführt.

In einer anderen bevorzugten Ausführungsform geht man von einer Verbindung VIII, worin $R_1'$ Cyano ist, aus und setzt diese in Gegenwart von Ammoniumchlorid mit etwa 90-99,5%igem, z.B. etwa 97-99%igen, wie etwa 98%igem, Methanol um. Dabei wird die Cyanogrupppe intermediär in die Methoxycarbiminogruppe überführt, die ohne Isolierung zu Methoxycarbonyl hydrolysiert wird.

Ein weiterer in Methoxycarbonyl überführbarer Rest $R_1'$ ist die Methoxymethylgruppe, die oxidativ, z.B. durch Behandeln mit einer Mangan-VII- oder Chrom-VI-Verbindung, z.B. mit Kaliumpermanganat oder Chromtrioxid in wässrigem Aceton oder wässrigem Pyridin, oder

mit Kaliumdichromat in Essigsäure oder verdünnter Schwefelsäure, in Methoxycarbonyl $R_1$ überführt werden kann. Man kann aber auch von einem Ausgangsstoff ausgehen, worin $R_1'$ Hydroxymethyl ist, und diesen in Gegenwart von Methanol oxidieren, beispielsweise durch Behandeln mit Mangandioxid in Methanol als Reaktionsmedium.

Ausgangsstoffe VIII, worin $R_1'$ gegebenenfalls verestertes Carboxy oder Cyano ist, werden beispielsweise in analoger Weise hergestellt, wie für Verbindungen I beschrieben, wobei man gemäss den Verfahrensvarianten a) bis e) oder g) von Ausgangsstoffen II, III, IV, VI, VII, XII oder IX, die statt der Methoxycarbonylgruppe eine der genannten Gruppen $R_1'$ aufweisen, ausgeht. Nachfolgend kann Cyano $R_1'$ durch Behandeln mit Methanol und einer Halogenwasserstoff-sowie, z.B. Brom- oder Chlorwasserstoffsäure, oder mit Schwefelsäure in Methoxycarbimino und freies Carboxy, z.B. durch Behandeln mit Thionylchlorid in Gegenwart von Pyrdin, in Halogencarbonyl überführt werden.

Verfahrensvariante g)

Der in Methoxy überführbare Rest $X_4$ ist beispielsweise Hydroxy, das durch Umsetzung mit einem Methylierungsmittel, wie einem Halogenwasserstoff-, Schwefel- oder Sulfonsäureester des Methanols, z.B. mit einem Methylhalogenid, wie Methylbromid oder Methyljodid, Dimethylsulfat oder einem Alkansulfonsäure-, gegebenenfalls substituierten Benzolsulfonsäure- oder Halogensulfonsäuremethylester, wie Methan-, Aethan-, Benzol-, p-Brombenzol-, p-Toluol- oder Fluorsulfonsäuremethylester, in Gegenwart einer Base, wie eines Alkalimetall- oder Erdalkalimetallhydroxides oder -carbonates, z.B. von Natrium- oder Kaliumhydroxid oder vor allem von Kaliumcarbonat in Acrylalkohol oder Aceton, wie Methoxy überführt werden kann. Gegen Methoxy austauschbare Reste $X_4$ sind beispielsweise Halogenatome, wie Chlor, Brom oder Jod, die durch Umsetzung mit einem Alkalimetall- oder Erdalkalimethanolat, z.B. mit Natriummethanolat, durch Methoxy ersetzt werden können.

Ausgangsstoffe IX werden beispielsweise erhalten, indem man ein entsprechendes 5-$X_4$-Tryptamin mit der annähernd doppeltmolaren Menge Acrylsäuremethylester kondensiert und das Reaktionsprodukt, z.B. in analoger Weise wie für die Verfahrensvariante b) angegeben in den entsprechenden 1-[(5-$X_4$-Indol-3-yl)ethyl]-1,2,5,6-tetrahydro-pyridin-3-carbonäuremethylester überführt.

Verfahrensgemäss erhältliche Verbindungen können in üblicher Weise in andere Verbindungen der Formel I überführt werden.

So können beispielsweise erhaltene freie salzbildende Verbindungen in an sich bekannter Weise in Salze überführt werden, z.B. durch Umsetzen einer Lösung der freien Verbindung in einem geeigneten Lösungsmittel oder Lösungsmittelgemisch mit einer entsprechenden Base bzw. Säure oder mit einem geeigneten Ionenaustauscher.

Erhaltene Salze können in an sich bekannter Weise in die freien Verbindungen umgewandelt werden, z.B. durch Behandeln mit einer Base, wie einem Alkalimetallhydroxid, einem Alkalimetallcarbonat oder -hydrogencarbonat, oder mit Ammoniak.

Erhaltene Salze können in an sich bekannter Weise in andere Salze überführt werden, Säureadditionssalze z.B. durch Behandlung eines Salzes einer organischen Säure mit einem geeigneten Metallsalz, wie einem Natrium-, Barium- oder Silbersalz, einer Säure in einem geeigneten Lösungsmittel, in welchen ein sich bildendes anorganisches Salz unlöslich ist und damit aus dem Reaktionsgemisch ausscheidet.

Die Verbindungen, einschliesslich ihre Salze, können auch in Form der Hydrate erhalten werden oder das zur Kristallisation verwendete Lösungsmittel einschliessen.

Infolge der engen Beziehung zwischen den neuen Verbindungen in freier Form und in Form ihrer Salze sind im vorausgegangenen und nachfolgend unter den freien Verbindungen oder ihren Salzen sinn-und zweckgemäss gegebenenfalls auch die entsprechenden Salze bzw. freien Verbindungen zu verstehen.

Die Erfindung betrifft auch diejenigen Ausführungsformen des Verfahrens, nach denen man von einer auf irgendeiner Stufe des Verfahrens als Zwischenprodukt erhältlichen Verbindungen ausgeht und die fehlenden Schritte durchführt oder einen Ausgangsstoff in Form eines Salzes verwendet oder insbesondere unter der Reaktionsbedingungen bildet.

So können Zwischenprodukte der Formeln III bzw. XIII gemäss der Verfahrensvariante b) in situ gebildet und ohne Isolierung weiterumgesetzt werden.

Neue Ausgangsstoffe, z.B. der Formeln II, III, IV, VIII und IX, die speziell für die Herstellung der erfindungsgemässen Verbindungen entwickelt wurden, insbesondere die zu den eingangs als bevorzugt gekennzeichneten Verbindungen der Formeln I führende Ausgangsstoffauswahl, die Verfahren zu ihrer Herstellung sowie ihre Verwendung als Zwischenprodukte, bilden ebenfalls einen Gegenstand der Erfindung.

Die neuen Verbindungen der Formel I können z.B. in Form pharmazeutischer Präparate Verwendung finden, welche eine therapeutisch wirksame Menge der Aktivsubstanz, gegebenenfalls zusammen mit anorganischen oder organischen, festen oder flüssigen, pharmazeutisch verwendbaren Trägerstoffen enthalten, die sich zur enteralen, z.B. oralen, oder parenteralen Verabreichung eignen. So verwendet man Tabletten oer Gelatinekapseln, welche den Wirkstoff zusammen mit Verdünnungsmitteln, z.B. Laktose, Dextrose, Saccharose, Mannitol, Sobitol, Cellulose und/oder Schmiermittel, z.B. Kieselerde, Talk, Stearinsäure oder Salze davon, wie Magnesium- oder Calciumstearat, und/oder Polyethylenglykol, aufweisen. Tabletten können ebenfalls

Bindemitel, z.B. Magnesiumaluminiumsilikat, Stärken, wie Mais-, Weizen-, Reis- oder Pfeilwurzstärke, Gelatine, Traganth, Methylcellulose, Natriumcarboxylmethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, oder Adsorptionsmittel Farbstoffe, Geschmackstoffe und Süssmittel aufweisen. Ferner kann man die neue Verbindung der Formel I in Form von parenteral verabreichbaren Präparaten oder von Infusionslösungen verwenden. Solche Lösungen sind vorzugsweise isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wirksame Stoffe enthalten können, werden in an sich bekannter Weise, z.B. mitels konventioeller Misch-, Granulier-, Dragier-, Lösungs- oder Lyophilisierungsverfahren, hergestellt und enthalten von etwa 0,1 % bis 100 %, insbesondere von etwa 1 % bis etwa 50 %, Lyophilisate bis zu 100 % des Aktivstoffes.

Die Erfindung betrifft ebenfalls die Verwendung der Verbindungen der Formel I, vorzugsweise in Form von pharmazeutischen Präparaten. Die Dosierung kann von verschiedenen Faktoren, wie Applikationsweise, Spezies, Alter und/oder individuellem Zustand abhängen. Die täglich zu verabreichenden Dosen liegen bei oraler Applikation zwischen etwa 5 und etwa 50 mg/kg und für Warmblüter mit einem Gewicht von etwa 70 kg vorzugsweise zwischen etwa 0,3 g und etwa 3,0 g.

Die nachfolgenden Beispiele dienen zur Illustration der Erfindung; Temperaturen sind in Celciusgraden, Drucke in mbar angegeben.

Beispiel 1: 22,4 g 1-Brom-2-(3-indolyl)-ethan, 22,2 g 1,2,5,6-Tetrahydro-pyridin-3-carbonsäuremethylester-hydrobromid (Guvacolinhydrobromid) und 39 g N-Ethyl-N,N-diisopropylamin werden unter Stickstoff in 750 ml Dimethylformamid gelöst und 16 Stunden gerührt. Dann engt man unter vermindertem Druck auf etwa 300 ml ein, fügt 500 ml Wasser hinzu und schüttelt dreimal mit je 250 ml Dichlormethan aus. Die organischen Phasen werden vereinigt und dreimal mit je 80 ml n-Salzsäure ausgeschüttelt. Die wässrigen Phasen werden vereinigt, mit 40%iger Natronlauge basisch gestellt und erneut dreimal mit je 100 ml Dichlormethan ausgeschüttelt. Die neuen organischen Phasen werden vereinigt, mit Wasser und anschliessend mit gesättigter Kochsalzlösung gewaschen, über Natriumsulfat getrocknet und zur Trockene eingedampft. Der Eindampfrückstand wird in 100 ml warmen Diethylethers gelöst und mit 150 ml n-Hexan versetzt. Nach dem Abkühlen kristallisiert der 1-[2-(Indol-3-yl)-ethyl]-1,2,5,6-tetrahydro-pyriin-3-carbonsäuremethylester aus, welcher abfiltriert und getrocknet wird; Smp. 76-77°, $^{1}H$-NMR-Spektrum (250 MHz, $CDCl_3$): Singulett (3H) bei $\delta$ = 3,76 pm.

Beispiel 2: 25,4 g 1-Brom-2-(5-methoxyindol-3-yl)-ethan, 22,2 g 1,2,5,6-Tetrahydropyridin-3-carbonsäuremethylester-hydrobromid (Guvacolinhydrobromid) und 39 g N-Ethyl-N,N-diisopropylamin werden unter Stickstoff in 750 ml Dimethylformamid gelöst und 16 Stunden gerührt. Dann engt man unter vermindertem Druck auf etwa 300 ml ein, fügt 500 ml Wasser hinzu und schüttelt dreimal mit je 250 ml Dichlormethan aus. Die organischen Phasen werden vereinigt und dreimal mit je 80 ml n-Salzsäure ausgeschüttelt. Die vereinigten wässrigen Phasen werden mit 40%iger Natronlauge basisch gestellt und erneut dreimal mit je 100 ml Dichlormethan ausgeschüttelt. Die organischen Phasen werden vereinigt, mit Wasser und anschliessend mit gesättigter Kochsalzlösung gewaschen, über Natriumsulfat getrocknet und zur Trockene eingedampft. Der Eindampfrückstand wird in 100 ml Dichlormethan gelöst und mit 200 ml Diethylether versetzt,

Nach dem Abkühlen kristallisiert der 1-[2-(5-Methoxyindol-3-yl)-ethyl]-1,2,5,6-tetrahydropyridin-3-carbonsäuremethylester aus, der abfiltriert und getrocknet wird; Smp. 156-158°.

Beispiel 3: 21,3 g 1-[2-(Indol-3-yl)-ethyl]-1,2,5,6-tetrahydropyridin-3-carbonsäuremethylester werden in 100 ml Methanol gelöst und unter Rühren mit 20 ml methanolischer Bromwasserstoffsäure (4n) versetzt. Dann fügt man 200 ml Diethylether hinzu. Es kristallisiert das Hydrobromid des 1-[2-(Indol-3-yl)-ethyl]-1,2,5,6-tetrahydro-pyridin-3-carbonsäuremethylesters vom Smp. 215-218° aus. In analoger Weise kann man auch das Hydrochlorid (Smp. 208-209°) und das Fumarat (Smp. 214 unter Zersetzung) herstellen.

Beispiel 4: 15,7 g 1-[2-(5-Methoxyindol-3-yl)-ethyl]-1,2,5,6-tetrahydropyridin-3-carbonsäuremethylester werden in 100 ml Methanol gelöst und unter Rühren mit 14 ml methanolischer Salzsäure (4n) versetzt. Man dampft zur Trockene ein, nimmt in 170 ml warmem Aceton auf, lässt abkühlen und filtriert. Man erhält das Hydrochlorid des 1-[2-(5-Methoxyindol-3-yl)-ethyl]-1,2,5,6-tetrahydropyridin-3-carbonsäuremethylesters vom Smp. 177-178°.

Beispiel 5: In eine Suspension von 6,5 g (18 mMol) 1-[2-(Indol-3-yl)ethyl]-3-methoxycarbonyl-pyridiniumbromid, in 43 ml Methanol werden unter Rühren bei -10° innerhalb von 90 Minuten 1,41 g Natriumborhydrid eingetragen. Man lässt 1 Stunde bei 0° und 2 Stunden bei Raumtemperatur nachrühren, versetzt mit 50 ml Wasser und schüttelt zweimal mit je 100 ml Dichlormethan aus. Die Dichlormethanphasen werden vereingt, über Magnesiumsulfat getrocknet und zur Trockne eingedampft. Das Rohprodukt wird an 150 g Kieselgel (0,063-0,2 mm) mit Essigsäureethylester als Laufmittel chromatographisch gereinigt und durch Behandeln des eingedampften Haupteluates mit etherischer Salzsäure in das 1-[2-(Indol-3-yl)ethyl]-1,2,5,6-tetrahydro-pyridin-3-carbonsäuremethylester-hydrochlorid vom Smp. 208-209° überführt.

Das Ausgangsmaterial kann z.B. folgendermassen hergestellt werden:

11,2 g (50 mMol) 2-(Indol-3-yl)-1-brom-ethan und 9,3 g (67,5 mMol) Nikotinsäuremethylester werden in 50 ml Butan-2-on suspendiert und 72 Stunden unter Rühren zum Sieden erhitzt. Man lässt abkühlen, saugt ab und trocknet unter vermindertem Druck. Das erhaltene 1-[2-(Indol-3-yl)ethyl]-3-methoxycarbonyl-pyridinium-bromid schmilzt bei 215-217°.

Beispiel 6: In einem 2,5 l fassenden Sulfierkolben mit Rührer, Tropftrichter und Thermometer werden 302,4 g Gemisch von cis- und trans-4-Hydroxy-1-[2-(indol-3-yl)ethyl]-piperidin-3-carbonsäuremethylester-hydrochlorid in 800 ml Pyridin gelöst, auf 0° bis 5° abgekühlt und unter Rühren bei 0° bis 5°tropfenweise mit 137,4 g Methansulfonylchlorid versetzt. Man rührt 4 Stunden bei Raumtemperatur, erwärmt auf 53° bis 56°, lässt 4 Stunden bei etwa 55° nachrühren und über Nacht stehen. Die Reaktionsmasse wird mit 800 ml Aceton verrührt, abgenutscht und mit Aceton nachgewaschen. Dann verrührt man mit 500 ml Acetonitril, kühlt auf 5° ab und nutscht erneut ab. Der erhaltene, rohe 1-[2-(Indol-3-yl)ethyl]-1,2,5,6-tetrahydro-pyridin-3-carbonsäuremethylester schmilzt bei 108-110°.Er wird aus Aceton umkristallisiert und schmilzt dann bei 109-110°.

Das Ausgangsmaterial kann beispielsweise folgendermassen hergestellt werden:

In einem 5 l fassenden Sulfierkolben mit Rührer, Thermometer und Tropftrichter werden unter Rühren 153 g Natriummethanolat in 1000 ml Dimethylformamid eingetragen. Dann wird innerhalb von etwa 12 Stunden eine Lösung von 665 g N-[2-(Indol-3-yl)ethyl]-imino-di(propionsäure)dimethylester in 680 ml Dimethylformamid zugetropft, wobei die Reaktionstemperatur auf etwa 40° steigt. Man lässt 3 Stunden bei Raumtemperatur nachrühren, destilliert unter vermindertem Druck bei höchstens 40° das Lösungsmittel ab, giesst in ein Gemisch von 2100 ml 5n-Salzsäure und 500 ml Toluol, nutscht ab, wäscht mit kaltem Methanol und trocknet bei 40° unter vermindertem Druck. Man erhält den 1-[2-(Indol-3-yl)ethyl]-piperidin-4-on-3carbonsäuremethylester-hydrochlorid bzw. 1-[2-(Indol-3-yl)ethyl]-4-hydroxy--1,2,5,6-tetrahydro-pyridin-3-carbonsäuremethylester-hydrochlorid vom Smp. 185-187°; Rf = 0,61 (Chloroform/Methanol 9:1).

In einem 6 l fassenden, mit Rührer, Thermometer und einem über einen weiten Gummischlauch angeschlossenen 250 ml-Rundkolben versehenen Sulfierkolben werden 337 g 1-[2-(Indol-3-yl)ethyl]-piperidin-4-on-3-carbonsäuremethylester-hydrochlorid in 3300 ml Methanol vorgelegt. Man lässt unter Rühren auf -15° abkühlen und trägt aus dem 250 ml-Rundkolben innerhalb von 2 Stunden bei -15 bis -5° portionsweise 68 g Natriumborhydrid ein. Man lässt eine Stunde bei °0 nachrühren, destilliert unter vermindertem Druck bei höchstens 40° Badtemperatur das Lösungsmittel ab, versetzt mit 1500 ml Essigsäureethylester, 1000 ml Eiswasser und 50 ml gesättigter Kaliumcarbonatlösung hinzu und lässt 30 Minuten rühren. Die organische Phase wird abgetrennt, über Magnesiumsulfat getrocknet und unter vermindertem Druck zur Trockne eingedampft. Man erhält ein Gemisch von cis- und trans-1-[2-(Indol-3-yl)ethyl]-4-hydroxy-piperidin-3-carbonsäuremethylester, Rf = 0,27 und 0,35 (Chloroform/Methanol 9:1).

Beispiel 7: In einem mit Rührer und Thermometer versehenen, 1,5 l fassenden Sulfierkolben werden 188 g 1-[2-(Indol-3-yl)ethyl]-1,2,5,6-tetrahydro-pyridin-3-carbonsäuremethylester unter Rühren in 710 ml Dichlormethan gelöst. Man versetzt mit 53 ml Methanol, kühlt auf 10° bis 15° ab und gibt langsam 144 ml etherische Salzsäure (etwa 17%ig G/V). Man lässt 15 Minuten nachrühren, nutscht ab, wäscht zweimal mit je 100 ml Dichlormethan und dann mit 500 ml Diethylether nach und trocknet 5 Stunden unter vermindertem Druck bei 50°. Man erhält das 1-[2-(Indol-3-yl)ethyl]-1,2,5,6-tetrahydro-pyridin-3-carbonsäuremethylester-hydrochlorid vom Smp. 210° (Zers.), Rf = 0.53 (Chloroform + Methanol + konz. Ammoniak; 9:1:1).

Beispiel 8: 2,7 g 1-[2-(5-Hydroxyindol-3-yl)ethyl]-1,2,5,6-tetrahydropyridin-3-carbonsäuremethylester-hydrobromid werden in 50 ml Aceton suspendiert, mit 5 g wasserfreiem Kaliumcarbonat und 0,9 g

Dimethylsulfat versetzt und 5 Stunden zum Sieden erhitzt. Man lässt abkühlen, filtriert, dampft das Filtrat zur Trockne ein und kristallisiert aus Dichlormethan/Diethylether um. Man erhält den 1-[2-(5-Methoxyindol-3-yl)ethyl]-1,2,5,6-tetrahydro-pyridin-3-carbonsäuremethylester vom Smp. 157-158°.

Das Ausgangsmaterial kann in analoger Weise wie in Beispiel 5 beschrieben ausgehend von 2-(5-Hydroxyindol-3-yl)-1-brom-ethan und Nikotinsäuremethylester über 1-[2-(5-Hydroxyindol-3-yl)ethyl]-3-methoxycarbonyl-pyridinimbromid erhalten werden.

Beispiel 9: In eine Suspension von 2,4 g Natriumhydrid (55 %ige Suspension in Mineralöl) in 50 ml Dimethylformamid tropft man eine Lösung von 14,22 g (0,05 Mol) 2-[2-(Indol-3-yl)ethyl]-aminomethyl-penta-2,4-dien-carbonsäuremethylester in 100 ml Dimethylformamid und rührt 3 Stunden bei Raumtemperatur. Man engt unter vermindertem Druck auf etwa 70 ml ein, fügt 150 ml Wasser hinzu und schüttelt dreimal mit je 100 ml Dichlormethan aus. Die organischen Phasen werden über Natriumsulfat getrocknet und das Lösungsmittel abgedampft. Der Rückstand wird in Methanol gelöst und mit methanolischer Salzsäure versetzt. Nach Zusatz von Diethylether kristallisiert beim Abkühlen 1-[2-(Indol-3-yl)ethyl]-1,2,5,6-tetrahydro-pyridin-3-carbonsäuremethylester-hydrochlorid vom Smp. 208-209°.

Das Ausgangsmaterial kann z.B. folgendermassen hergestellt werden:

Eine gut gerührte Lösung von 31,5 g (0,25 Mol) 2-Methyl-penta-2,4-dien-carbonsäuremethylester (hergestellt nach J.L. Belletire und D.R. Walley: Tetrahedron Letters 1983, 1475), 44,5 g N-Bromsuccinimid und 200 mg Azobisisobutyronitril in 200 ml Tetrachlormethan wird 12 Stunden zum Rückfluss erhitzt, wobei eine Lösung mit einer 200 W Lampe bestrahlt wird. Nach dem Erkalten filtriert man vom ausgefallenen Succinimid ab, wäscht den Filterrückstand 2x mit je 30 ml Tetrachlorkohlenstoff nach, extrahiert die vereinigten organischen Phasen mit Natriumcarbonatlösung und Wasser, trocknet über Natriumsulfat und dampft das Lösungsmittel im Vakuum ab. Man

erhält 2-Brommethyl-penta-2,4-dien-carbonsäuremethylester als orangerotes Oel. Eine Lösung von 16 g 1-Amino-2-(indol-3-yl)-ethan, 20,5 g (0,1 Mol) 2-Brommethyl-penta-2,4-dien-carbonsäuremethylester und 39 g N-Ethyl-N,n-Diisopropyl-amin in 500 ml Dimethylformamid wird unter Stickstoff 16 Stunden bei Raumtemperatur gerührt. Man engt unter vermindertem Druck auf etwa 250 ml ein, fügt 500 ml Wasser hinzu und schüttelt dreimal mit je 250 ml Dichlormethan aus. Die organischen Phasen werden vereinigt, über Natriumsulfat getrocknet und eingedampft. Nach Reinigung durch Chromatographie an Kieselgel mit Toluol/Ethylacetat (9:1) als Laufmittel erhält man 2-[2-(1H-Indol-3-yl)ethyl]-aminomethyl-penta-2,4-dien-carbonsäuremethylester als gelbes Oel.

Beispiel 10: Man versetzt eine siedene Lösung von 4,32 g (40 mMol) von Phenylhydrazin in 110 ml Methanol und 10 ml Wasser mit einer Lösung von 8,45 g (40 mMol) 4-[1-(3-Methoxycarbonyl-1,2,5,6-tetrahydro)-pyridyl]-butanal in 50 ml Methanol und erhitzt 20 Stunden zum Rückfluss. Das Lösungsmittel wird am Vakuum abgedampft, der Rückstand in heisser 0,5%-iger Salzsäurelösung gelöst und über 1 g Aktivkohle filtriert. Die Lösung wird konzentriert und der Rückstand aus Ethanol/Diethylether umkristallisiert. Man erhält so 1-[2-(1H-indol-3-yl)ethyl]-1,2,5,6-tetrahydro-pyridin-3-carbonsäuremethylester-hydrochlorid vom Smp. 208-209 (Zers.).

Das Ausgangsmaterial kann z.B. folgendermassen hergestellt werden:

15,5 g 4-Chlor-1,1-dimethoxy-butan, 22,2 g 1,2,5,6-Tetrahydro-pyridin-3-carbonsäuremethylester-hydrobromid (Guvacolin-hydrobromid) und 39 g N-Ethyl-N,N-diisopropyl-amin werden unter Stickstoff in 750 ml Dimethylformamid gelöst und 16 Stunden bei 50° gerührt. Man engt unter vermindertem Druck auf etwa 300 ml ein, fügt 500 ml Wasser hinzu und schüttelt dreimal mit je 250 ml Dichlormethan aus. Die organischen Phasen werden vereinigt, über Natriumsulfat getrocknet und im Vakuum eingedampft. Man erhält so 4-[1-(3-Methoxycarbonyl-1,2,5,6-tetrahydro)-pyridyl]-1,1-dimethoxy-butan als gelbes Oel.

Aktivkohle filtriert. Die Lösung wird konzentriert und der Rückstand aus Ethanol/Diethylether umkristallisiert. Man erhält so 1-[2-(Indol-3-yl)ethyl]-1,2,5,6-tetrahydro-pyridin-3-carbonsäuremethylester-hydrochlorid vom Smp. 208-209 (Zers.).

Das Ausgangsmaterial kann z.B. folgendermassen hergestellt werden:

15,5 g 4-Chlor-1,1-dimethoxy-butan, 22,2 g 1,2,5,6-Tetrahydro-pyridin-3-carbonsäuremethylester-hydrobromid (Guvacolin-hydrobromid) und 39 g N-Ethyl-N,N-diisopropyl-amin werden unter Stickstoff in 750 ml Dimethylformamid gelöst und 16 Stunden bei 50° gerührt. Man engt unter vermindertem Druck auf etwa 300 ml ein, fügt 500 ml Wasser hinzu und schüttelt dreimal mit je 250 ml Dichlormethan aus. Die organischen Phasen werden vereinigt, über Natriumsulfat getrocknet und im Vakuum eingedampft. Man erhält so 4-[1-(3-Methoxycarbonyl-1,2,5,6-tetrahydro)-pyridyl]-1,1-dimethoxy-butan als gelbes Oel.

Eine Lösung von 18 g (70 mMol) von 4-[1-(3-Methoxycarbonyl-1,2,5,6-tetrahydro)-pyridyl]-1,1-dimethoxy-butan in 200 ml Dichlormethan wird mit 100 g Kieselgel, welches nach J.M. Conia et al., Synthesis 1978, Seite 63, mit 10%-iger wässriger Oxalsäure imprägniert ist, versetzt und die Suspension 3 Stunden bei Raumtemperatur gerührt. Nach Abfiltrieren wäscht man mit 5%-iger wässriger Natriumbicarbonatlösung, trocknet die organische Phase mit Natriumsulfat und dampft zur Trockne ein. Man erhält so 4-[1-(3-Methoxycarbonyl-1,2,5,6-tetrahydro)-pyridyl]-butanal als gelbes Oel.

Beispiel 11: Eine Lösung von 6 g (20 mMol) 1-[2-(Indol-3-yl)ethyl]-1,2,5,6-tetrahydro-pyridin-3-carbonsäureethylester in 100 ml Methanol wird mit 2,5 ml konzentrierter Schwefelsäure versetzt und während 16 Stunden zum Rückfluss erhitzt. Nach Erkalten wird das Lösungsmittel im Vakuum abgedampft, der Rückstand in 50 ml Wasser aufgenommen und mit gesättigter Natriumbicarbonatlösung behandelt. Die nunmehr neutrale Lösung wird dreimal mit je 100 ml Dichlormethan extrahiert, die vereinigten organischen Phasen über Natriumsulfat

153 g Natriumethanolat werden unter Rühren in 1000 ml Dimethylformamid gelöst. Dann tropft man innerhalb von 90 Minuten eine Lösung von 721 g (2 Mol) N-[2-(1H-Indol-3-yl)ethyl]-imino-di(3-propionsäure)diethylester in 680 ml Dimethylformamid hinzu, erwärmt auf 40°, lässt 3 Stunden bei Raumtemperatur nachrühren und destilliert das Lösungsmittel bei 40° unter vermindertem Druck ab. Der Rückstand wird bei 20 bis 35° in ein Gemisch von 2100 ml 5n-Salzsäure und 500 ml Toluol eingegossen. Das ausgefallene 4-Hydroxy-1-[2-(1H-indol-3-yl)ethyl]-1,2,5,6-tetrahydropyridin-3-carbonsäureethylester-hydrochlorid bzw. 1-[2-(1H-Indol-3-yl)ethyl]-piperidin-4-on-3-carbonsäureethylester-hydrochlorid wird abgenutscht, mit kaltem Methanol gewaschen und bei 40° unter vermindertem Druck getrocknet. Es schmilzt bei 170-173° (Zers.).

In einem 5 l fassenden, mit Rührer, Thermometer und einem über einen weiten Gummischlauch angeschlossenen 250 ml-Rundkolben versehenen Sulfierkolben werden 294 g 1-[2-(Indol-3-yl)ethyl]-piperidin-4-on-3-carbonsäureethylester-hydrochlorid in 2750 ml Methanol vorgelegt. Man lässt unter Rühren auf -15° abkühlen und trägt aus dem 250 ml-Rundkolben innerhalb von 2 Stunden bei -15 bis -5° portionsweise 56,7 g Natriumborhydrid ein. Man lässt eine Stunde bei °0 nachrühren, destilliert unter vermindertem Druck bei höchstens 40° Badtemperatur das Lösungsmittel ab, versetzt mit 1250 ml Essigsäureethylester, 800 ml Eiswasser und 50 ml gesättigter Kaliumcarbonatlösung hinzu und lässt 30 Minuten rühren. Die organische Phase wird abgetrennt, über Magnesiumsulfat getrocknet und unter vermindertem Druck zur Trockne eingedampft. Man erhält ein Gemisch von cis- und trans-1-[2-(Indol-3-yl)ethyl]-4-hydroxy-piperidin-3-carbonsäureethylester, das ohne weitere Reinigung verwendet werden kann.

In einem 2,5 l fassenden Sulfierkolben mit Rührer, Tropftrichter und Thermometer werden 237,3 g Gemisch von cis- und trans-1-[2-(Indol-3--yl)ethyl]-4-hydroxy-piperidin-3-carbonsäureethylester-hydrochlorid in 600 ml Pyridin gelöst, auf 0° bis 5° abgekühlt und unter Rühren bei 0° bis 5° tropfenweise mit 103,1 g Methansulfonylchlorid

versetzt. Man rührt 4 Stunden bei Raumtemperatur, erwärmt auf 53° bis 56°, lässt 4 Stunden bei etwa 55° nachrühren und über Nacht stehen. Die Reaktionsmasse wird mit 800 ml Aceton verrührt, abgenutscht und mit Aceton nachgewaschen. Dann verrührt man mit 500 ml Acetonitril, kühlt auf 5° ab und nutscht erneut ab. Der erhaltene, rohe 1-[2-(Indol-3-yl)ethyl]-1,2,5,6-tetrahydro-pyridin-3-carbonsäureethylester vom Smp. 151-155° kann ohne weitere Reinigung verwendet werden.

Beispiel 12: Eine Lösung von 10 g (0.04 Mol) von 1-[2-(Indol-3-yl)-ethyl]-1,2,5,6-tetrahydro-pyridin-3-carbonsäurenitril in 200 ml 95%igem Methanol wird mit 4 ml konzentrierter Schwefelsäure versetzt und 16 Stunden zum Rückfluss erhitzt. Nach dem Erkalten wird unter vermindertem Druck auf 50 ml eingeengt, der Rückstand in 200 ml Dichlormethan und 100 ml Wasser verteilt und mit gesättigter Natriumbicarbonatlösung neutralisiert. Die organische Phase wird abgetrennt und die wässrige Phase noch einmal mit 100 ml Dichlormethan nachgeschüttelt.

Die vereinigten organischen Phasen werden über Natriumsulfat getrocknet und eingedampft. Nach Reinigung durch Chromatographie an 400 g Kieselgel mit Toluol/Ethylacetat (9:1) als Laufmittel erhält man nach Kristallisation aus Diethylether/n-Hexan 1-[2-(Indol-3-yl)-ethyl]-1,2,5,6-tetrahydro-pyridin-3-carbonsäuremethylester vom Smp. 75-77°.

Das Ausgangsmaterial kann man z.B. folgendermassen herstellen: 22,4 g 1-Brom-2-(3-indolyl)-ethan (0,1 Mol), 10,8 g 1,2,5,6-Tetrahydropyridin-3-carbonsäurenitril und 20 g N-Ethyl-N,N-diisopropylamin werden unter Stickstoff in 450 ml Dimethylformamid gelöst und 16 Stunden gerührt. Dann engt man unter vermindertem Druck auf 150 ml ein, fügt 500 ml Wasser hinzu und schüttelt dreimal mit je 150 ml Dichlormethan aus. Die organischen Phasen werden vereinigt und dreimal mit je 75 ml n-Salzsäure ausgeschüttelt. Die wässrigen Phasen werden vereinigt, mit 40 % Natronlauge basisch gestellt und erneut mit dreimal 100 ml Dichlormethan ausgeschüttelt. Die organi-Die vereinigten organischen Phasen werden über Natriumsulfat getrocknet und eingedampft. Nach Reinigung durch Chromatographie an 400 g Kieselgel mit Toluol/Ethylacetat (9:1) als Laufmittel erhält man nach Kristallisation aus Diethylether/n-Hexan 1-[2-(Indol-3-yl)-ethyl]-1,2,5,6-tetrahydro-pyridin-3-carbonsäuremethylester vom Smp. 75-77°.

Das Ausgangsmaterial kann man z.B. folgendermassen herstellen: 22,4 g 1-Brom-2-(3-indolyl)-ethan (0,1 Mol), 10,8 g 1,2,5,6-Tetrahydropyridin-3-carbonsäurenitril und 20 g N-Ethyl-N,N-diisopropylamin werden unter Stickstoff in 450 ml Dimethylformamid gelöst und 16 Stunden gerührt. Dann engt man unter vermindertem Druck auf 150 ml ein, fügt 500 ml Wasser hinzu und schüttelt dreimal mit je 150 ml Dichlormethan aus. Die organischen Phasen werden vereinigt und dreimal mit je 75 ml n-Salzsäure ausgeschüttelt. Die wässrigen Phasen werden vereinigt, mit 40 % Natronlauge basisch gestellt und erneut mit dreimal 100 ml Dichlormethan ausgeschüttelt. Die organischen Phasen werden vereingt, über Natriumsulfat getrocknet und zur Trockne eingedampft. Man erhält so 1-[2-(Indol-3-yl)ethyl]-1,2,5,6-tetrahydro-pyridin-3-carbonsäurenitril als gelbes Oel. Smp. des Hydrochlorids:

Beispiel 13: Man versetzt eine Lösung von 15,36 g (60 mMol) 3-Hydroxymethyl-1-[2-(1H-indol-3-yl)ethyl]-1,2,5,6-tetrahydro-pyridin in 1000 ml Methanol unter Kühlung portionsweise mit 15 g Natriumcyanid, 150 g aktiviertem Mangandioxid sowie 6 ml Eisessig und rührt die Suspension in einer Stickstoffatmosphäre 18 Stunden bei Raumtemperatur. Nach Abfiltrieren wird das Filtrat zur Trockne eingedampft, der Rückstand in 300 ml Methylenchlorid aufgenommen und diese Lösung dreimal mit je 100 ml Wasser extrahiert. Nach Reinigung durch Chromatographie an 500 g Kieselgel mit Toluol/Ethylacetat (9:1) als Laufmittel erhält man nach Kristallisation aus Diethylether/n-Hexan 1-[2-(Indol-3-yl)ethyl]-1,2,5,6-tetrahydro-pyridin-3-carbonsäuremethylester vom Smp. 76-77°.

Beispiel 14: Man löst portionsweise in einer Stickstoffatmosphäre 4,82 g metallisches Natrium in 60 ml trockenem Methanol, versetzt diese Lösung mit 100 ml Dimethylformamid, 10 g Kupfer(I)-iodid und 7,26 g (0,02 Mol) 1-[2-(5-Brom-indol-3-yl)ethyl]-1,2,5,6-tetrahydropyridin-3-carbonsäuremethylester und erhitzt 16 Stunden auf 130°. Nach Abkühlen und Filtration versetzt man mit 300 ml Ethylacetat und extrahiert dreimal mit je 150 ml Wasser. Die organische Phase wird über Natriumsulfat getrocknet und eingedampft. Nach Reinigung durch Chromatographie an 300 g Kieselgel mit Toluol/Ethylacetat (9:1) als Laufmittel erhält man nach Kristallisation aus Dichlormethan/Diethylether 1-[2-(5-Methoxy-indol-3-yl)ethyl]-1,2,5,6-tetrahydro-pyridin-3-carbonsäuremethylester vom Smp. 157-158°.

Das Ausgangsmaterial kann in analoger Weise wie im Beispiel 5 beschrieben ausgehend von 2-(5-Bromindol-3-yl)-1-brom-ethan, erhältlich durch Behandeln von 2-(5-Bromindol-3-yl)ethanol mit Brom und Triphenylphosphin in Acetonitril und Nikotin-säuremethylester über 1-[2-(5-Bromindol-3-yl)ethyl]-3-methoxycarbonyl-pyridinium-bromid erhalten werden.

Beispiel 15: Eine Lösung von 7,66 g (25 mMol) 1-[2-(Indol-3-yl)-ethyl]-1,2,5,6-tetrahydro-pyridin-3-carbonsäure-hydrochlorid in 150 ml Methanol wird mit 1 ml konzentrierter Schwefelsäure versetzt und 4 Stunden zum Rückfluss erhitzt. Nach Erkalten wird die Lösung am Vakuum eingedampft, der Rückstand mit je 100 ml Dichlormethan und Wasser versetzt und mit wässriger Natriumcarbonatlösung neutralisiert. Die wässrige Phase wird abgetrennt und erneut 3-mal mit je 50 ml Dichlormethan extrahiert. Die vereinigten organischen Phasen werden über Natriumsulfat getrocknet und unter vermindertem Druck eingedampft. Analog Beispiel 3 wird mit methanolischer Bromwasserstoffsäure (4n) 1-[2-(Indol-3-yl)ethyl]-1,2,5,6-tetrahydro-pyridin-3-carbonsäuremethylester-hydrobromid vom Smp. 215-218° erhalten.

Das Ausgangsmaterial kann z.B. folgendermassen hergestellt werden:

Man versetzt eine Lösung von 12,8 g (0,05 Mol) 3-Hydroxymethyl-1-[2-(indol-3-yl)ethyl]-1,2,5,6-tetrahydro-pyridin in 150 ml Tetrahydrofuran mit einer Lösung von 2,5 g Natriumhydroxid in 80 ml Wasser und mit 40 g Nickelperoxidtrihydrat und rührt 8 Stunden in einer Stickstoffatmosphäre bei 50°. Nach dem Erkalten wird die Lösung filtriert und im Vakuum auf ca. 100 ml eingeengt. Nach dreimaliger Extraktion mit je 70 ml Dichloräthan wird die wässrige Phase mit 1n Salzsäure angesäuert und erneut dreimal mit je 100 ml Dichlormethan ausgeschüttelt. Die vereinigten organischen Phasen werden über Natriumsulfat getrocknet und im Vakuum eingedampft. Durch Versetzen mit methanolischer Salzsäure und Abkühlen erhält man 1-[2-(Indol-3-yl)ethyl]-1,2,5,6-tetrahydro-pyridin-3-carbonsäure-hydrochlorid vom Smp. 170-171°.

Beispiel 16: Tabletten, enthaltend je 50 mg 1-[2-(Indol-3-yl)-ethyl]-1,2,5,6-tetrahydro-pyridin-3-carbonsäuremethylester bzw. dessen Hydrochlorid, können wie folgt hergestellt werden:

| Zusammensetzung (1000 Tabletten) | |
|---|---|
| Wirkstoff | 500.0 g |
| Lactose | 500.0 g |
| Kartoffelstärke | 352.0 g |
| Gelatine | 8.0 g |
| Talk | 60.0 g |
| Magnesiumstearat | 10.0 g |
| Siliciumdioxid (hochdisper.) | 20.0 g |
| Ethanol | q.s. |

Der Wirkstoff wird mit der Lactose und 292 g Kartoffelstärke vermischt, die Mischung mit einer alkoholischen Lösung der Gelatine befeuchtet und durch ein Sieb granuliert. Nach dem Trocknen mischt man den Rest der Kartoffelstärke, den Talk, das Magnesiumstearat und das hochdisperse Siliciumdioxid zu und presst das Gemisch zu

Tabletten von je 145,0 mg Gewicht und 50,0 mg Wirkstoffgehalt, die gewünschtenfalls mit Teilkerben zur feineren Anpassung der Dosierung versehen sein können.

Beispiel 17: Lacktabletten, enthaltend je 100 mg 1-[2-(Indol-3-yl)-ethyl]-1,2,5,6-tetrahydro-pyridin-3-carbonsäuremethylester, können wie folgt hergestellt werden:

| Zusammensetzung (für 1000 Tabletten) | |
|---|---|
| Wirkstoff | 100.00 g |
| Lactose | 100.00 g |
| Maisstärke | 70.00 g |
| Talk | 8.50 g |
| Calciumstearat | 1.50 g |
| Hydroxypropyl-methylcellulose | 2.36 g |
| Schellack | 0.64 g |
| Wasser | q.s. |
| Methylenchlorid | q.s. |

Der Wirkstoff, die Lactose und 40 g der Maisstärke werden gemischt und mit einem Kleister, hergestellt aus 15 g Maisstärke und Wasser (unter Erwärmen), befeuchtet und granuliert. Das Granulat wird getrocknet, der Rest der Maisstärke, der Talk und das Calciumstearat werden zugegeben und mit dem Granulat vermischt. Das Gemisch wird zu Tabletten (Gewicht: 280 mg) verpresst und diese mit einer Lösung der Hydroxypropylmethylcellulose und des Schellacks in Methylenchlorid lackiert; Endgewicht der Lacktablette: 283 g.

Beispiel 18: In analoger Weise wie in den Beispielen 16 und 17 beschrieben kann man auch pharmazeutische Präparate, enthaltend eine andere Verbindung der Formel I gemäss den Beispielen 1 bis 15 herstellen.

Patentansprüche für die Vertragsstaaten: DE, GB, FR, CH, LI, IT, NL, BE, SE, LU

1. Ein 1-(3-Indolyl)-tetrahydropyridin3-carbonsäureester der Formel

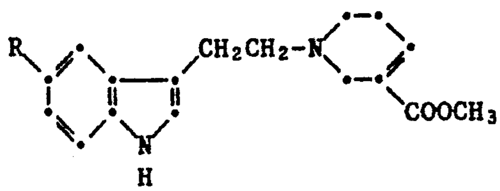

(I)

worin R Wasserstoff oder Methoxy bedeutet, oder ein Salz davon.

2. 1-[2-(Indolyl-3-yl)-ethyl]-1,2,5,6-tetrahydro-pyridin-3-carbonsäuremethylester oder ein Säureadditionssalz davon.

3. 1-[2-(5-Methoxyindol-3-yl)-ethyl]-1,2,5,6-tetrahydropyridin-3-carbonsäuremethylester oder ein Säureadditionssalz davon.

4. Eine Verbindung gemäss einem der Ansprüche 1-3 in Form des Hydrochlorides oder Hydrobromides.

5. Eine Verbindung gemäss einem der Ansprüche 1-4 zur Anwendung in einem Verfahren zur therapeutischen Behandlung des menschlichen oder tierischen Körpers.

6. Eine Verbindung gemäss einem der Ansprüche 1-4 als Nootropikum.

7. Pharmazeutische Präparate enthaltend eine Verbindung gemäss einem der Ansprüche 1-4 neben üblichen pharmazeutischen Hilfs-und/oder Trägerstoffen.

8. Verwendung einer Verbindung gemäss einem der Ansprüche 1-4 zur Herstellung eines Arzneimittels.

9. Verfahren zur Herstellung neuer 1-(3-Indolyl)-tetrahydropoyridine der Formel

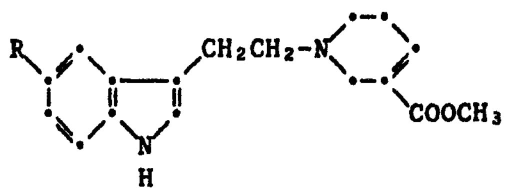

(I)

worin R Wasserstoff oder Methoxy bedeutet, und ihrer Salze, dadurch gekennzeichnet, dass man

a) in einer Verbindung der Formel

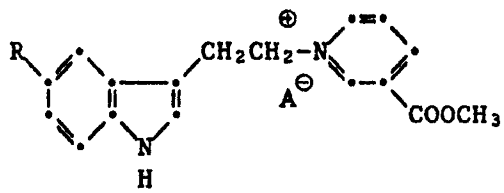

(II),

worin $A^{\ominus}$ ein Säureanion bedeutet, die überschüssigen Doppelbindungen zu Einfachbindungen reduziert oder

b) aus einer Verbindung der Formel

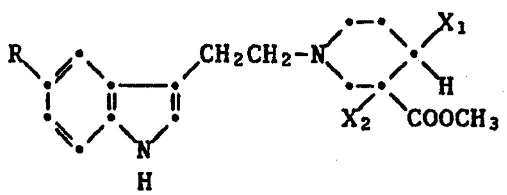

(III),

worin einer der Reste $X_1$ und $X_2$ Wasserstoff und der andere einen abspaltbaren Rest bedeutet, oder einem Salz davon, $X_1$ und $X_2$ unter Bildung der Doppelbindung abspaltet, oder

c) eine Verbindung der Formel

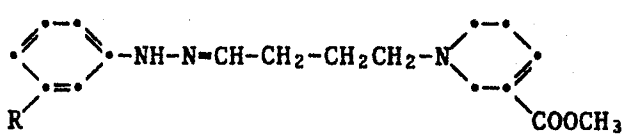

(IV)

oder ein Salz davon zu der entsprechenden Verbindung der Formel I ringschliesst, oder

d) Verbindungen der Formeln

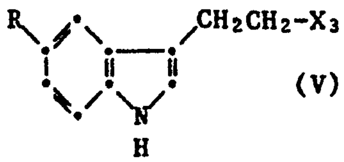 (V) und 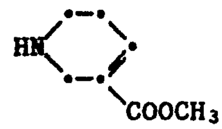 (VI),

worin $X_3$ eine nukleofuge Abgangsgruppe bedeutet, oder deren Salze miteinander umsetzt, oder

e) eine Verbindung der Formel

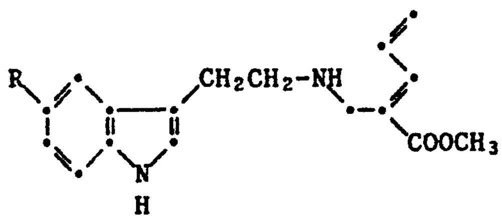

(VII),

oder ein Salz davon cyclisiert, oder

f) in einer Verbindung der Formel

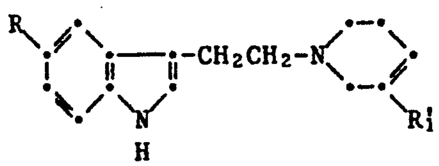

(VIII),

worin $R_1'$ einen in Methoxycarbonyl überführbaren Rest bedeutet, oder in einem Salz davon $R_1'$ in Methoxycarbonyl überführt, oder

g) zur Herstellung der Verbindung der Formel I, worin R Methoxy ist, in einer Verbindung der Formel

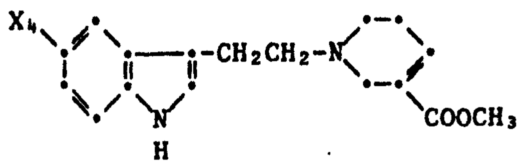

(IX),

worin $X_4$ einen in Methoxy überführbaren oder gegen Methoxy austauschbaren Rest bedeutet, $X_4$ in Methoxy überführt oder gegen Methoxy austauscht und gewünschtenfalls die verfahrensgemäss erhältliche Verbindung in eine andere Verbindung der Formel I überführt, ein verfahrensgemäss erhältliches Isomerengemisch in die Komponenten auftrennt und/oder eine verfahrensgemäss erhältliche freie Verbindung in ein Salz oder ein verfahrensgemäss erhältliches Salz in die freie Verbindung oder in ein anderes Salz umwandelt.

10. Verfahren gemäss Anspruch 9, dadurch gekennzeichnet, dass man

a) in einer Verbindung der Formel

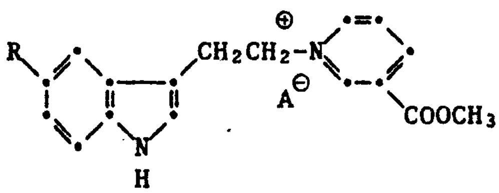

(II),

worin $A^{\ominus}$ ein Säureanion bedeutet, die überschüssigen Doppelbindungen zu Einfachbindungen reduziert oder

b) aus einer Verbindung der Formel

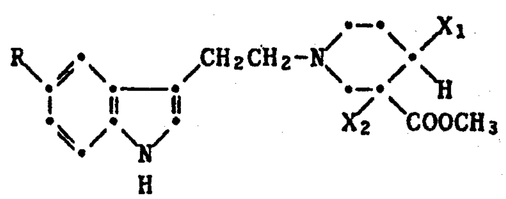

(III),

worin einer der Reste $X_1$ und $X_2$ Wasserstoff und der andere einen abspaltbaren Rest bedeutet, oder einem Salz davon, $X_1$ und $X_2$ unter Bildung der Doppelbindung abspaltet, oder

c) eine Verbindung der Formel

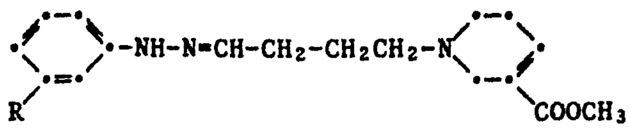

(IV)

oder ein Salz davon zu der entsprechenden Verbindung der Formel I ringschliesst, oder

d) Verbindungen der Formeln

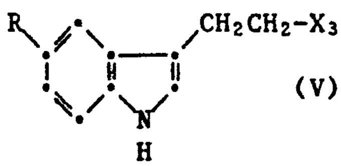

(V) und

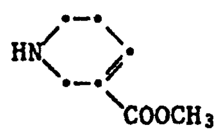

(VI),

worin $X_3$ eine nukleofuge Abgangsgruppe bedeutet, oder deren Salze miteinander umsetzt, oder

e) eine Verbindung der Formel

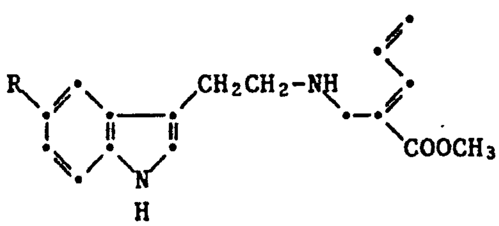

(VII),

oder ein Salz davon cyclisiert und gewünschtenfalls die verfahrensgemäss erhältliche Verbindung in eine andere Verbindung der Formel I überführt, ein verfahrensgemäss erhältliches Isomerengemisch in die reinen Komponenten auftrennt und/oder eine verfahrensgemäss erhältliche freie Verbindung in ein Salz oder ein verfahrensgemäss erhältliches Salz in die freie Verbindung oder in ein anderes Salz umwandelt.

11. Die nach dem Verfahren des Anspruches 9 oder 10 erhältlichen neuen Verbindungen.

FO 7.4/KVB/eg*

Patentansprüche für den Vertragsstaat AT

1. Verfahren zur Herstellung neuer 1-(3-Indolyl)-tetrahydropoyridine der Formel

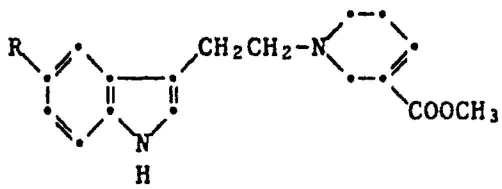

(I)

worin R Wasserstoff oder Methoxy bedeutet, und ihrer Salze, dadurch gekennzeichnet, dass man

a) in einer Verbindung der Formel

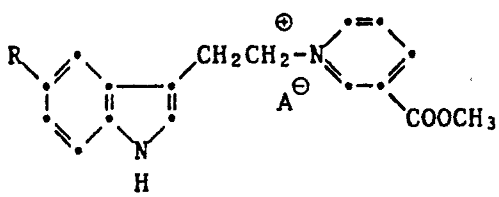

(II),

worin $A^{\ominus}$ ein Säureanion bedeutet, die überschüssigen Doppelbindungen zu Einfachbindungen reduziert oder

b) aus einer Verbindung der Formel

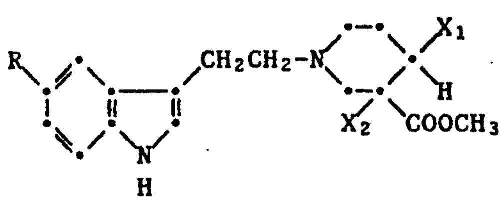

(III),

worin einer der Reste $X_1$ und $X_2$ Wasserstoff und der andere einen abspaltbaren Rest bedeutet, oder einem Salz davon, $X_1$ und $X_2$ unter Bildung der Doppelbindung abspaltet, oder

c) eine Verbindung der Formel

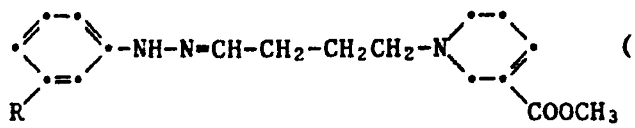

(IV)

oder ein Salz davon zu der entsprechenden Verbindung der Formel I ringschliesst, oder

d) Verbindungen der Formeln

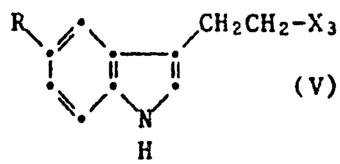

(V) und

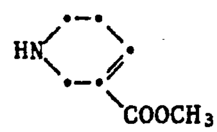

(VI),

worin $X_3$ eine nukleofuge Abgangsgruppe bedeutet, oder deren Salze miteinander umsetzt, oder

e) eine Verbindung der Formel

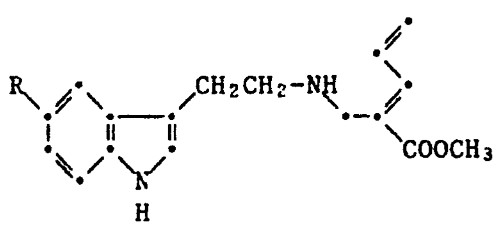

(VII),

oder ein Salz davon cyclisiert, oder

f) in einer Verbindung der Formel

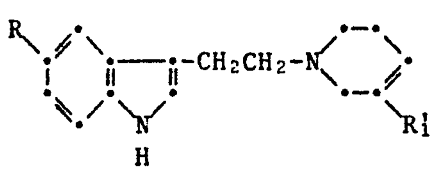

(VIII),

worin $R_1^{\prime}$ einen in Methoxycarbonyl überführbaren Rest bedeutet, oder in einem Salz davon $R_1^{\prime}$ in Methoxycarbonyl überführt, oder

0187619

g) zur Herstellung der Verbindung der Formel I, worin R Methoxy ist, in einer Verbindung der Formel

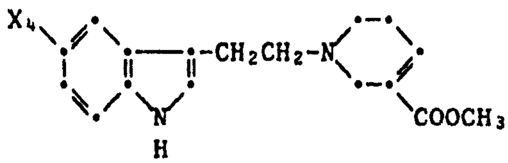

(IX),

worin $X_4$ einen in Methoxy überführbaren oder gegen Methoxy austauschbaren Rest bedeutet, $X_4$ in Methoxy überführt oder gegen Methoxy austauscht und gewünschtenfalls die verfahrensgemäss erhältliche Verbindung in eine andere Verbindung der Formel I überführt, ein verfahrensgemäss erhältliches Isomerengemisch in die Komponenten auftrennt und/oder eine verfahrensgemäss erhältliche freie Verbindung in ein Salz oder ein verfahrensgemäss erhältliches Salz in die freie Verbindung oder in ein anderes Salz umwandet.

2. Verfahren gemäss Anspruch 1, dadurch gekennzeichnet, dass man 1-[2-(Indolyl-3-yl)-ethyl]-1,2,5,6-tetrahydro-pyridin-3-carbonsäuremethylester oder ein Säureadditionssalz davon herstellt.

3. Verfahren gemäss Anspruch 1, dadurch gekennzeichnet, dass man 1-[2-(5-Methoxyindol-3-yl)-ethyl]-1,2,5,6-tetrahydropyridin-3-carbonsäuremethylester oder ein Säureadditionssalz davon herstellt.

4. Verfahren gemäss Anspruch 1, dadurch gekennzeichnet, dass man eine Verbindung, erhältlich gemäss einem der Ansprüche 1-3, in Form des Hydrochlorides oder Hydrobromides herstellt.

5. Verfahren zur Herstellung pharmazeutischer Präparate, dadurch gekennzeichnet, dass man eine Verbindung, erhältlich gemäss einem der Ansprüche 1-4, mit üblichen pharmazeutischen Hilfs- und/oder Trägerstoffen vermischt.

FO 7.4/KVB/eg*